# EUROPÄISCHE PATENTANMELDUNG

(11) **EP 4 310 011 A1**
(43) Veröffentlichungstag der Anmeldung: **24.01.2024**
(21) Anmeldenummer: 22186286.5
(22) Anmeldetag: 21.07.2022
(51) Int. Cl.: B65B 5/06, B32B 27/36, B65B 5/02, C12M 1/22

(54) **VERWENDUNG EINER POLYMERFOLIE ZUM VERPACKEN EINES BEHÄLTERS**

(71) Anmelder: SÜDPACK Medica AG, 6340 Baar (CH)
(72) Erfinder: Hermann, Michael, 5400 Baden (CH); Grimbacher, Carolin, 88416 Ochsenhausen (DE)
(74) Vertreter: Christ, Niko

(57) **Zusammenfassung**

Die Erfindung betrifft die Verwendung einer Polymerfolie zum Verpacken wenigstens eines Behälters, bevorzugt einer Petrischale, die ein Mikroorganismen-Kulturmedium in gelierter Form enthält.

Aus dem Stand der Technik ist die Verwendung von Cellophan für die Umverpackung von Petrischalen, aufgrund der sterilen Barriere bei gleichzeitiger hoher Wasserdampfdurchlässigkeit, vorbekannt. Allerdings ist Cellophan in der Herstellung ressourcen- und energieintensiv, sodass sich die hier beschriebene Erfindung die Aufgabe stellt, eine kostengünstigere Alternative bereitzustellen, mit vergleichbaren Materialeigenschaften.

Dies gelingt der Erfindung dadurch, dass die Polymerfolie eine oder mehrere Polylactid-Schichten umfasst, eine Wasserdampfdurchlässigkeit von 80 g/m² x 24 Stunden bis 380 g/m² x 24 Stunden aufweist, und direkt durch Extrusion oder Kaschierung erhalten wird.

## Beschreibung

Die Erfindung betrifft die Verwendung einer Polymerfolie zum Verpacken wenigstens eines Behälters, vorzugsweise wenigstens einer Petrischale, die ein Mikroorganismen-Kulturmedium in gelierter Form enthält.

Petrischalen mit Mikroorganismen-Kulturmedien, insbesondere solche mit Agar-Agar, werden insbesondere in der Mikrobiologie genutzt, um Bakterienkulturen zu züchten. Um allerdings Fremdbakterien und -viren sowie sonstige Umwelteinflüsse auszuschließen und dennoch ein optimales Klima für die Aufzucht der gewünschten Bakterien zu schaffen, werden die angeimpften Agar-Agar-Platten meist in einer Verpackung aus Cellophan eingeschweißt bzw. eingeschlossen. Denn mit einer Verpackung aus Cellophan wird eine sterile Barriere gegenüber der Umwelt erreicht und darüber hinaus aufgrund der niedrigen Wasserdampfbarriere des Cellophans das Auftreten von etwaigem Kondenswasser vermieden. Allerdings ist Cellophan gegenüber anderen Kunststoffen relativ teuer, da dieses vorwiegend aus nachwachsenden Rohstoffen gewonnen werden muss und somit bei der Herstellung ein hoher Energie- und Ressourcenaufwand besteht.

Daher hat es sich die hier beschriebene Erfindung zur Aufgabe gemacht, eine Polymerfolie zum Verpacken wenigstens eines Behälters, vorzugsweise wenigstens einer Petrischale, die ein Mikroorganismen-Kulturmedium in gelierter Form enthält, insbesondere Agar-Agar, bereitzustellen, die ebenfalls eine sterile Barriere schafft und eine zu Cellophan vergleichbare niedrige Wasserdampfbarriere aufweist, um der Bildung von Kondenswasser innerhalb der Verpackung entgegenwirken zu können.

Diese Aufgabe wird durch die Verwendung einer Polymerfolie gemäß dem geltenden Anspruch 1 gelöst. Vorteilhafte Ausgestaltungen können den abhängigen Ansprüchen entnommen werden.

Erfindungsgemäß handelt es sich um die Verwendung einer Polymerfolie zum Verpacken wenigstens eines Behälters, vorzugsweise wenigstens einer Petrischale, die ein Mikroorganismen-Kulturmedium in gelierter Form enthält. Die Erfindung sieht zur Lösung der Aufgabe vor, dass die Polymerfolie eine oder mehrere Polylactid-Schichten umfasst, einer Wasserdampfdurchlässigkeit von 80 g/m² × 24 Stunden bis 380 g/m² × 24 Stunden aufweist, und direkt durch Extrusion oder Kaschierung erhalten wird.

Durch die Verwendung von einer oder mehrerer Polylactid-Schichten können die Anforderungen an die Verpackung für wenigstens einen Behälter, bevorzugt eine Petrischale sowohl an die sterile Barriere als auch an die hohe Wasserdampfdurchlässigkeit erfüllt werden. Ferner ist eine Polymerfolie, die aus einer oder mehreren Polylactid-Schichten aufgebaut ist, kostengünstiger in der Herstellung als eine vergleichbare Polymerfolie aus Cellophan.

Um einer möglichen Kondensatbildung ferner entgegenwirken zu können, sieht die erfindungsgemäße Verwendung in einer vorteilhaften Ausgestaltung vor, dass eine oder mehrere der Polylactid-Schichten und/oder eine andere Kunststoff-Schicht der Polymerfolie mikroperforiert ist. Somit kann die Wasserdampfdurchlässigkeit der Polymerfolie weiter verbessert werden.

Damit bei der Verarbeitung bzw. Herstellung eines Beutels für eine Verpackung wenigstens einer Petrischale eine Rissbildung der Polymerfolie vermieden werden kann, sieht die Verwendung in einer weiteren vorteilhaften Ausgestaltung vor, dass zwischen wenigstens einer ersten und zweiten Polylactid-Schicht und/oder einer anderen Kunststoff-Schicht der Polymerfolie ein Gleitmittel eingebracht wird. Besonders bevorzugt ist der Anteil des Gleitmittels dabei zwischen 2 bis 5 Gew.-%.

Vorteilhafterweise weist die Polymerfolie eine Schichtdicke von 10 µm bis 100 µm, bevorzugt von 20 µm bis 40 µm, besonders bevorzugt von 20 µm.

Um die Vernetzung der Polylactid-Schichten und/oder anderen KunststoffSchichten bzw. der Polymerfolie insgesamt zu erhöhen und/oder um die Polymerfolie zu sterilisieren, sieht die Erfindung in einer weiteren vorteilhaften Ausgestaltung vor, dass die Polymerfolie mit einer ionisierten Strahlung behandelt wird. Die Energiedosis der ionisierten Strahlung richtet sich dabei gewöhnlich nach der Durchdringungsrate, also wie tief die ionisierte Strahlung in die Polymerfolie eindringt, der Einwirkungszeit, also der Dauer der Strahlung an einer Position der Polymerfolie, sowie der Behandlungsanzahl. Daher kann es beispielsweise bei besonders dünnen Folien sinnvoll sein, mit einer geringen Intensität, jedoch mit mehreren Zyklen die Polymerfolie zu behandeln, um die gewünschte Vernetzung bzw. Sterilität zu erhalten, ohne dabei die Polymerfolie anzugreifen bzw. die Polymere der Polymerfolie zu zerstören. Bevorzugt liegt die Energiedosis dabei in einem Bereich von 25 kGy bis 100 kGy, besonders bevorzugt von 75 kGy und 100 kGy.

Um dem Alterungsprozess der Polymerfolie, insbesondere die durch die ionisierte Strahlung hervorgerufene Alterung zu vermeiden, sieht die Erfindung in einer weiteren vorteilhaften Ausgestaltung vor, dass die Polymerfolie ein Additiv zum Alterungsschutz enthält, insbesondere ein Radikalfänger oder Säurefänger. Somit kann die Langlebigkeit der Polymerfolie weiter gesteigert werden.

Letztlich sieht die Erfindung in einer weiteren vorteilhaften Ausgestaltung vor, dass die erfindungsgemäße Polymerfolie für die Herstellung eines Beutels für die Verpackung wenigstens einer Petrischale, welche bevorzugt ein Mikroorganismen-Kulturmedium aufweist, verwendet wird.

Somit ist vorstehend die Verwendung einer kostengünstigen alternativen Polymerfolie zum Verpacken wenigstens einer Petrischale offenbart, mit der vergleichbare Materialeigenschaften wie der des Cellophans gewährleistet werden können.

## Patentansprüche

1. Verwendung einer Polymerfolie zum Verpacken wenigstens eines Behälters, vorzugsweise einer Petrischale, die ein Mikroorganismen-Kulturmedium in gelierter Form enthält,
**dadurch gekennzeichnet, dass** die Polymerfolie
- eine oder mehrere Polylactid-Schichten umfasst,
- eine Wasserdampfdurchlässigkeit von 80 g/m² × 24 Stunden bis 380 g/m² × 24 Stunden aufweist, und
- direkt durch Extrusion oder Kaschierung erhalten wird.

2. Verwendung nach Anspruch 1, **dadurch gekennzeichnet, dass** eine oder mehrere der Polylactid-Schichten und/oder eine andere Kunststoff-Schicht der Polymerfolie mikroperforiert ist.

3. Verwendung nach einem der Ansprüche 1 oder 2, **dadurch gekennzeichnet, dass** zwischen wenigstens einer ersten und einer zweiten Polylactid-Schicht und/oder einer anderen Kunststoff-Schicht der Polymerfolie ein Gleitmittel eingebracht ist.

4. Verwendung nach Anspruch 3, **dadurch gekennzeichnet, dass** der Anteil des Gleitmittels 2 bis 5 Gew.-% beträgt.

5. Verwendung nach wenigstens einem der vorhergehenden Ansprüche 1 bis 4, **dadurch gekennzeichnet, dass** die Polymerfolie eine Dicke zwischen 10 µm und 100 µm, vorzugsweise 20 µm bis 40 µm, besonders bevorzugt von 20 µm, aufweist.

6. Verwendung nach wenigstens einem der vorhergehenden Ansprüche 1 bis 5, **dadurch gekennzeichnet, dass** die Polymerfolie mit einer ionisierten Strahlung behandelt wird.

7. Verwendung nach wenigstens einem der vorhergehenden Ansprüche, **dadurch gekennzeichnet, dass** die Polymerfolie ein Additiv zum Alterungsschutz enthält, insbesondere ein Radikalfänger oder Säurefänger.

8. Verwendung einer Polymerfolie nach wenigstens einem der vorhergehenden Ansprüche 1 bis 6 zur Herstellung eines Beutels für die Verpackung wenigstens eines Behälters, vorzugsweise einer Petrischale.
